# EUROPÄISCHE PATENTSCHRIFT

(11) **EP 2 827 775 B1**
(45) Veröffentlichungstag und Bekanntmachung des Hinweises auf die Patenterteilung: **18.04.2018**
(21) Anmeldenummer: 13706427.5
(22) Anmeldetag: 18.02.2013
(51) Int. Cl.: A61B 8/00, A61B 5/00, G01S 15/89

(54) **ULTRASCHALL-MESSEINRICHTUNG, UNTERSUCHUNGSGERÄT UND VERFAHREN ZU DEREN BETRIEB**
ULTRASONIC MEASURING DEVICE, EXAMINATION APPARATUS AND METHOD FOR OPERATING SAME
DISPOSITIF DE MESURE À ULTRASONS, APPAREIL D'ANALYSE ET PROCÉDÉ SERVANT À FAIRE FONCTIONNER LEDIT DISPOSITIF ET LEDIT APPAREIL

(30) Priorität: 23.03.2012 DE 102012005895
(43) Veröffentlichungstag der Anmeldung: 28.01.2015
(73) Patentinhaber: Fraunhofer-Gesellschaft zur Förderung der angewandten Forschung e.V., 80686 München (DE)
(72) Erfinder: TRETBAR, Steffen, 66125 Saarbrücken (DE); DEGEL, Christian, 66440 Niederwürzbach (DE); GÜNTHER, Matthias, 28359 Bremen (DE)
(74) Vertreter: v. Bezold & Partner Patentanwälte - PartG mbB
(86) Internationale Anmeldenummer: PCT/EP2013/000470
(87) Internationale Veröffentlichungsnummer: WO 2013/139422

(56) Entgegenhaltungen:
- WO-A1-2004/064642
- WO-A2-2008/042559
- JP-A- 2006 230 912
- US-A1- 2007 167 705
- US-A1- 2010 152 590
- US-A1- 2010 168 577
- US-B2- 7 945 304

## Beschreibung

Die Erfindung betrifft eine Ultraschall-Messeinrichtung mit einem Ultraschall-Array zur Erfassung von Ultraschallsignalen, das in einem Gehäuse angeordnet ist, insbesondere für bildgebende Verfahren oder Verfahren zur Ultraschall-basierten Messung von Probeneigenschaften. Die Ultraschall-Messeinrichtung zeichnet sich insbesondere durch ein Ultra-schall-Array aus, das in dem Gehäuse mit einer Stelleinrichtung verstellbar sein kann. Des Weiteren betrifft die Erfindung ein Untersuchungsgerät, das eine oder mehrere derartige Ultra-schall-Messeinrichtungen enthält. Des Weiteren betrifft die Erfindung Verfahren zur Ultraschall-Signalerfassung unter Verwendung der Ultraschall-Messeinrichtung, wie z. B. bildgebende Verfahren oder Verfahren zur Ultraschall-basierten Messung von Probeneigenschaften. Anwendungen der Erfindung sind insbesondere in der medizinischen Bildgebung, in der Ultra-schall-basierten Probenbehandlung und der zerstörungsfreien Untersuchung von Proben gegeben.

Die Verwendung von manuell positionierten Ultraschall-Messköpfen in der medizinischen Bildgebung ist allgemein bekannt. Ein Ultraschall-Messkopf wird vom Anwender (Arzt) manuell auf den gewünschten Körperabschnitt aufgesetzt und dort ggf. bewegt, um eine optimale Position für die Bildgebung zu finden. Wenn die Bildgebung für Beobachtungszwecke wiederholt werden soll, muss der Ultraschall-Messkopf auf dem Körperabschnitt fixiert werden. Aus der Praxis ist bekannt, einen Ultra-schall-Messkopf mit einem mechanischen Haltearm zu fixieren. Diese Fixierung ist jedoch statisch und in der Regel ungeeignet, bei Bewegungen des zu untersuchenden Objektes (z. B. durch Atmung, Herzschlag, usw.) zuverlässige und vergleichbare Ultraschallbilder zu liefern. Der gewünschte zu untersuchende Bereich kann sich aus dem durch die Fixierung definierten, statischen Bilderfassungsbereich des Ultraschall-Messkopfes herausbewegen, so dass eine Auswertung der ermittelten Ultraschallbilder beeinträchtigt ist.

Aus US 5 598 845 A ist ein Ultraschall-Messkopf bekannt, der mit einem Klebeband auf einem zu untersuchenden Bereich eines Körperabschnitts positionierbar ist. Der herkömmliche Ultra-schall-Messkopf umfasst ein Ultraschall-Array, das in einem Gehäuse angeordnet ist. Durch einen Schallfensterabschnitt des Gehäuses können Ultraschallwellen vom Ultraschall-Array in den zu untersuchenden Bereich eingekoppelt und nach Rückreflektion mit dem Ultraschall-Array erfasst werden. Das Gehäuse sitzt in einem Kragen, der mit dem Klebeband, das neben dem Schallfensterabschnitt angeordnet ist, auf dem zu untersuchenden Bereich fixierbar ist. Die Ausrichtung des Gehäuses gemeinsam mit dem Ultraschall-Array relativ zum Kragen ist verstellbar, um das Schallfeld des Ultraschall-Arrays auf einen bestimmten zu untersuchenden Bereich zu richten.

Der herkömmliche Ultraschall-Messkopf gemäß US 5 598 845 A hat die folgenden Nachteile. Erstens führt das Klebeband zu Problemen beim Positionieren des Ultraschall-Messkopfes. Beispielsweise kann sich das Klebeband falten und an sich selbst oder an Teilen des Ultraschall-Messkopfes anhaften. Des Weiteren muss auf der Oberfläche des untersuchten Körperabschnitts zusätzlich zum Schallfensterabschnitt auch für das Klebeband genügend Platz vorhanden sein. Ein weiterer Nachteil besteht darin, dass das Ultraschall-Array nur gemeinsam mit dem Gehäuse verstellbar. Dies bedeutet, dass die Verstellung zu einer Bewegung des Gehäuses relativ zum Körperabschnitt und damit zu einer Beeinträchtigung der Fixierung führt. Des Weiteren stellt die Zusammensetzung aus dem Gehäuse und dem Kragen einen komplexen Aufbau dar, der teuer ist und einen hohen Herstellungsaufwand erfordert. Wenn der Ultraschall-Messkopf an verschiedenen Patienten verwendet werden soll, erfordert dies einen hohen Reinigungsaufwand, der durch den komplexen Aufbau noch vergrößert wird. Ein weiteres Problem besteht in der Verstellung des Gehäuses relativ zum Kragen. Für eine Verstellung müssen Stifte, die von der Oberfläche des Gehäuses durch den Kragen nach außen ragen, bewegt werden. Die Verstellung erfordert ein manuelles Eingreifen des Anwenders.

Der herkömmliche Ultraschall-Messkopf ist daher für Anwendungen nur beschränkt geeignet, bei denen auch über längere Zeitintervalle der zu untersuchende Bereich permanent und zuverlässig erfasst werden soll, wie z.B. bei der Ultraschall-Bildgebung, bei der Bewegungskorrektur mittels Ultraschall, zur effektiven Steuerung von Radiotherapiesystemen, für Ultraschall-Therapiesysteme oder andere positionsgesteuerte Interventionen.

Ein weiteres generelles Problem der herkömmlichen Ultraschalltechnik ist bei der multimodalen Bildgebung und bei der Steuerung von Therapiesystemen gegeben, wenn der Betrieb des Ultraschall-Messkopfs die komplementäre Modalität oder das Therapieverfahren stört oder umgekehrt durch die Modalität oder das Therapieverfahren gestört wird. Beispielsweise besteht ein Interesse, die Ultraschall-Bildgebung mit der MRT-(Magnetresonanz-Tomographie)-Bildgebung zu kombinieren, wobei jedoch herkömmliche Ultraschall-Messköpfe den Betrieb des MR-Tomographen stören und durch die Hochfrequenzfelder im MR-Tomographen gestört werden. Gegenwärtig ist kein Ultraschall-Messkopf bekannt, der für einen Einsatz im MRT-Gerät oder während einer Radiotherapie geeignet wäre. WO2004/064642 offenbart eine Ultraschall-Messeinrichtung umfassend ein Gehäuse mit einem Schallfensterabschnitt, der für ein Anhaften auf einer Oberfläche des zu untersuchenden Gegenstands konfiguriert ist.

Aus US2007/167705 und US7945304 ist die Abschirmung von Ultraschall Geräten für die Benutzung innerhalb eines Kernspintomographen. US2010/152590 offenbart die Verwendung eines piezoelektrischen Stellmotors in einem katheter-basierten Ultraschallgerät.

Die genannten Probleme betreffen nicht nur Ultraschallmessungen, bei denen Ultraschallsignale durch die Einkopplung von Ultraschall in den zu untersuchenden Bereich erzeugt werden. Die Probleme treten z. B. auch bei Ultraschallmessungen auf, bei denen die Ultraschallsignale durch Einkopplung von gepulstem Licht angeregt werden (photoakustische Bildgebung). Die Aufgabe der Erfindung ist es, eine verbesserte Ultra-schall-Messeinrichtung bereitzustellen, mit der Nachteile herkömmlicher Techniken überwunden werden und die insbesondere für eine dauerhafte und zuverlässige Fixierung auf einem zu untersuchenden Gegenstand, eine zuverlässige Verstellung im fixierten Zustand, eine verminderte Empfindlichkeit gegenüber äußeren Störungen und/oder einen verminderten Störeinfluss in einer Umgebung der Messeinrichtung geeignet ist. Eine weitere Aufgabe der Erfindung ist es, ein verbessertes Untersuchungsgerät bereitzustellen, das mit mindestens einer derartigen Ultraschall-Messeinrichtung ausgestattet ist. Des Weiteren ist es eine Aufgabe der Erfindung, ein verbessertes Verfahren zur Ultraschallmessung bereitzustellen, mit dem Nachteile herkömmlicher Techniken vermieden werden und das insbesondere für eine dauerhafte und zuverlässige, positionsgenaue und/oder störungs-minimierte Messung geeignet ist.

Diese Aufgaben werden durch eine Ultraschall-Messeinrichtung mit den Merkmalen des unabhängigen Anspruchs gelöst. Vorteilhafte Ausführungsformen und Anwendungen der Erfindung ergeben sich aus den abhängigen Ansprüchen.

Gemäß einem ersten Gesichtspunkt der Offenbarung wird die oben genannte Aufgabe durch eine Ultraschall-Messeinrichtung gelöst, die ein Gehäuse umfasst, in dem ein Ultraschall-Array angeordnet ist. Das Ultraschall-Array umfasst mindestens zwei Ultraschall-Wandlerelemente, mit denen Ultraschallsignale erfassbar sind. Das Ultraschall-Array weist eine vorbestimmte Empfindlichkeitscharakteristik auf, die einen an das Ultra-schall-Array angrenzenden Raumbereich bildet, in dem die Ultraschallsignale in gerichteter Weise erfassbar sind bzw. in den bei Betrieb des Ultraschall-Arrays als Emitter Ultraschallwellen gerichtet abgestrahlt werden. Die Empfindlichkeitscharakteristik hängt insbesondere von der Position und/oder Orientierung des Ultraschall-Arrays relativ zu dem zu untersuchenden Gegenstand ab.

Das Gehäuse umfasst allgemein ein Bauteil mit einem ein- oder mehrteiligen Innenraum, in dem erfindungsgemäß das Ultra-schall-Array angeordnet ist. Das Gehäuse umfasst eine Gehäusewand und einen Schallfensterabschnitt. Der Schallfensterabschnitt hat typischerweise eine Gestalt einer ebenen oder gewölbten, schichtförmigen Wand, die für eine Übertragung von Ultraschallwellen angepasst ist. Der Schallfensterabschnitt ist ein Teil des Gehäuses, durch das Ultraschall vom Ultra-schall-Array gerichtet in die Umgebung abgestrahlt und/oder empfangen wird. Das Ultraschall-Array ist in dem Gehäuse in akustischem Kontakt mit dem Schallfensterabschnitt angeordnet.

Gemäß der Offenbarung ist der Schallfensterabschnitt für ein Anhaften auf einer Oberfläche des zu untersuchenden Gegenstandes eingerichtet. Die äußere Oberfläche des Schallfensterabschnitts ist mit anhaftendem Material versehen. Der Schallfensterabschnitt trägt auf der Außenseite des Gehäuses mindestens eine Klebefläche. Der Schallfensterabschnitt hat eine Haftfähigkeit derart, dass das Gehäuse mit den in diesem angeordneten Komponenten der Ultraschall-Messeinrichtung zuverlässig auf der Oberfläche des zu untersuchenden Gegenstands zuverlässig fixierbar ist. Die Erfinder haben festgestellt, dass Ultraschallwellen durch den Schallfensterabschnitt mit dem anhaftenden Material in den zu untersuchenden Gegenstand einkoppelbar und aus diesem auskoppelbar sind. Vorzugsweise ist ein selbstklebender Schallfensterabschnitt vorgesehen. Das anhaftende Material dient sowohl der Befestigung der Ultraschall-Messeinrichtung als auch der Schallleitung. Ein zusätzliches Schall-Übertragungsmedium ist auf der Außenseite des Gehäuses nicht erforderlich. Vorteilhafterweise wird gleichzeitig die Fixierung der Ultraschall-Messeinrichtung auf dem Gegenstand und die Übertragung von Ultraschallsignalen durch den Schallfensterabschnitt realisiert. Die Nachteile von Klebestreifen werden vermieden. Die Ultraschall-Messeinrichtung erhält einen kompakten Aufbau.

Gemäß einer bevorzugten Ausführungsform der Offenbarung kann der Schallfensterabschnitt vom übrigen Gehäuse trennbar angeordnet sein. Der Schallfensterabschnitt ist mit der Gehäusewand lösbar verbunden. Zur Schallübertragung kann zwischen den trennbaren Teilen ein Gel, Öl oder Fett vorgesehen werden. Dies ermöglicht vorteilhafterweise einen einfachen Austausch des Schallfensterabschnitts, z.B. wenn die Ultra-schall-Messeinrichtung zu einer neuen Anwendung überführt werden soll. Besonders bevorzugt ist vorgesehen, dass der Schallfensterabschnitt Eingriffselemente aufweist, welche zur lösbaren Befestigung an der Gehäusewand eingerichtet sind. Die Eingriffselemente sind seitlich vom Schallfensterabschnitt abstehende Vorsprünge, die mit passenden Aufnahmen an der Gehäusewand zusammenwirken. Vorteilhafterweise ermöglichen die Eingriffselemente eine einfache Entfernung des Schallfensterabschnitts vom Gehäuse, z.B. für Austausch- oder Reinigungszwecke.

Vorteilhafterweise kann gemäß einer weiteren Ausführungsform der Erfindung der Schallfensterabschnitt ein Einwegprodukt sein. Der Schallfensterabschnitt ist zum einmaligen Gebrauch mit einem bestimmten Gegenstand, wie z.B. einem bestimmten Probanden, und/oder einer bestimmten Position auf der Oberfläche des zu untersuchenden Gegenstands eingerichtet. Vorteilhafterweise werden umständliche Reinigungs- oder Vorbereitungsschritte bei Anwendung der Ultraschall-Messeinrichtung bei einem neuen Gegenstand und/oder einer neuen Position auf dem Gegenstand vermieden.

Gemäß einer besonders bevorzugten Ausführungsform der Offenbarung hat es sich beispielsweise als ausreichend erwiesen, wenn der Schallfensterabschnitt aus mindestens einer Kunststofffolie hergestellt ist. Besonders bevorzugt ist die mindestens eine Kunststofffolie mit einem Abschirmmaterial beschichtet. Die Kunststofffolie kann eine Schicht aus einem Material tragen, das für eine elektro-magnetische Abschirmung geeignet ist. Des weiteren kann die mindestens eine Folie eine selbstklebende Oberfläche aufweisen, d. h. dass mindestens eine Folie eine selbstklebende Folie ist. Der Schallfensterabschnitt kann insbesondere aus zwei oder mehr Folien zusammengesetzt sein. Beispielsweise kann eine erste, innere Folie das Abschirmmaterial tragen und eine zweite, äußere Folie eine selbstklebende Folie umfassen.

Als vorteilhaft hat sich ferner erwiesen, wenn das Abschirmmaterial z. B. eine eingebrachte elektrisch leitende Folie oder ein elektrisch leitendes Netz, leitende Lacke oder Klebstoffe oder eine Metall-Sputterschicht, z. B. eine Zink-, Aluminium-, Gold-, Kupfer-, oder Titan-Sputterschicht, umfasst. Die Erfinder haben festgestellt, dass z. B. eine Sputterschicht mit einer Dicke von mindestens 10 nm und/oder höchstens 1000 µm, geeignet ist, die Abschirmfunktion zu erfüllen, ohne die Übertragung von Ultraschallsignalen zu beeinträchtigen.

Gemäß einer Variante der Offenbarung kann das Ultraschall-Array mit dem Gehäuse fest verbunden sein. Vorteilhafterweise ergibt sich in diesem Fall ein besonders einfacher Aufbau der Ultraschall-Messeinrichtung. Die Richtung der Empfindlichkeitscharakteristik des Ultraschall-Arrays kann durch die Position und/oder Orientierung der Ultraschall-Messeinrichtung bei deren Positionierung auf dem Gegenstand und/oder durch eine gezielte Ansteuerung von Wandlerelementen des Ultra-schall-Arrays eingestellt werden.

Gemäß einer bevorzugten Ausführungsform der Offenbarung ist vorgesehen, dass das Ultraschall-Array mit einer Stelleinrichtung relativ zum Gehäuse, insbesondere relativ zum Schallfensterabschnitt beweglich ist. Das Ultraschall-Array kann im Gehäuse bewegt, z. B. gedreht oder geschwenkt, werden, während die Gehäusewand und der Schallfensterabschnitt unbeweglich bleiben. Im Unterschied zu dem herkömmlichen Ultraschall-Messkopf gemäß US 5 598 845 A, bei dem das Ultra-schall-Array nur gemeinsam mit dem Gehäuse beweglich ist, bietet diese Ausführungsform Erfindung die Möglichkeit einer zuverlässigen Fixierung der Ultraschall-Messeinrichtung auf dem zu untersuchenden Gegenstand. Die Fixierung wird durch die Verstellung des Ultraschall-Arrays nicht beeinträchtigt, da das Gehäuse und insbesondere der Schallfensterabschnitt, der den Gegenstand berührt, bei einer Verstellung des Ultra-schall-Arrays unbeweglich bleiben.

Gemäß einer besonders bevorzugten Ausführungsform der Offenbarung ist die Ultraschall-Messeinrichtung mit der elektrisch, mechanisch, hydraulisch oder pneumatisch betätigbaren Stelleinrichtung ausgestattet, die zur Einstellung und/oder Bewegung des Ultraschall-Arrays relativ zu einem zu untersuchenden Gegenstand eingerichtet ist. Die Stelleinrichtung ist konfiguriert, das Ultraschall-Array in Bezug auf den zu untersuchenden Gegenstand einzustellen und/oder zu bewegen. Mit der Stelleinrichtung ist die Bewegung, Position und/oder Orientierung des Ultraschall-Arrays relativ zu dem Gegenstand einstellbar.

Des Weiteren ist gemäß dieser Ausführungsform der Offenbarung vorgesehen, dass die Stelleinrichtung im Gehäuse angeordnet ist. Die Gehäusewand kann einstückig gebildet sein, so dass sie den gesamten Innenraum einschließt, oder sie kann mehrteilig gebildet sein, so dass in einem Teil des Innenraums die Stelleinrichtung und in einem weiteren Teil des Innenraums das Ultraschall-Array angeordnet sind. Die Aufnahme der Stelleinrichtung im Gehäuse der Ultraschall-Messeinrichtung ergibt vorteilhafterweise einen kompakten Aufbau der Ultra-schall-Messeinrichtung. Im Unterschied zum Ultraschall-Messkopf gemäß US 5 598 845 A, bei dem das Gehäuse mit dem Ultraschall-Array manuell oder mit einer separaten Antriebseinheit verstellt werden muss, ist die Ultraschall-Messeinrichtung ein kompaktes Bauteil, dessen Funktionen (Erfassung von Ultraschallsignalen, ggf. Abstrahlung von Ultraschallwellen, Orientierung des Ultraschall-Arrays relativ zum zu untersuchenden Gegenstand) vollständig zum Beispiel über eine Verbindungsleitung elektrisch steuerbar sind. Während der Anwendung der Ultraschall-Messeinrichtung, insbesondere im fixierten Zustand an einem zu untersuchenden Gegenstand, ist die Ultraschall-Messeinrichtung bei Verstellung des Ultraschall-Arrays keinen mechanischen Kräften ausgesetzt, welche die Fixierung auf den Gegenstand beeinträchtigen würden. Der kompakte Aufbau ermöglicht des Weiteren, dass Störungen, die sich von der Ultraschall-Messeinrichtung auf ein weiteres Gerät, wie z.B. ein MRT-Gerät, auswirken könnten, minimiert oder vollständig unterdrückt werden können.

Gemäß einer besonders bevorzugten Ausführungsform der Offenbarung hat das Gehäuse eine Doppelfunktion. Erstens bildet es die oben genannte mechanische Halterung für das Ultraschall-Array und die Stelleinrichtung. Zweitens bildet es eine Verkapselung des Ultraschall-Arrays und der Stelleinrichtung, insbesondere eine elektro-magnetische Abschirmung. Vorzugsweise ist die elektro-magnetische Abschirmung eine Barriere für elektro-magnetische Felder in der Umgebung der Ultra-schall-Messeinrichtung. Des Weiteren bildet die Abschirmung eine Barriere für elektro-magnetische Felder, welche im Innenraum des Gehäuses generiert werden und den Betrieb eines weiteren Geräts stören könnten. Besonders bevorzugt ist die elektro-magnetische Abschirmung so konfiguriert, dass sie gegen Felder in einem Magnetresonanz-Tomographen wirksam ist, der z. B. mit einem statischen Magnetfeld von mindestens 1,5 T, z.B. 3 T, 7 T oder 12 T ausgelegt ist.

Die elektro-magnetische Abschirmung hat sich als besonders effektiv erwiesen, wenn mindestens eine der folgenden Maßnahmen vorgesehen ist. Gemäß einer ersten Variante kann eine Doppelschirmung des Ultraschall-Arrays und der Stelleinrichtung vorgesehen sein. Die Doppelschirmung umfasste einen ersten, auf Massepotential befindlichen Schirm und einen zweiten Schirm zur Dämpfung von elektro-magnetischen Feldern aus der Umgebung der Ultraschall-Messeinrichtung. Zweitens kann alternativ oder zusätzlich eine Mantelwellensperre vorgesehen sein, mit der elektro-magnetische Wellen auf der Verbindungsleitung so abgedämpft werden, dass keine Emission von elektro-magnetischen Feldern in die Umgebung erfolgt. Vorteilhafterweise wird eine eventuelle Antennenwirkung der Ultraschall-Messeinrichtung unterdrückt. Die Doppelschirmung hat insbesondere den Vorteil, dass die äußere Abschirmung unabhängig von der inneren Abschirmung des Ultraschall-Arrays auf das Potential im Feld z.B. eines MRT-Gerätes gelegt werden kann. Vorteilhafterweise kann damit eine unerwünschte Beeinflussung des Feldes im MR-Raum vermieden werden.

Die Stelleinrichtung der Ultraschall-Messeinrichtung umfasst allgemein einen elektrisch betätigbaren Antrieb, mit dem das Ultraschall-Array verbunden ist. Es ist beispielsweise ein elektrisch betätigbarer Motor vorgesehen, mit dem das Ultraschall-Array über eine Antriebswelle dreh- oder verschwenkbar ist. Gemäß einer besonders bevorzugten Ausführungsform der Erfindung umfasst die Stelleinrichtung einen piezoelektrischen Ringmotor, mit dem das Ultra-schall-Array relativ zum Schallfensterabschnitt beweglich ist. Der piezoelektrische Ringmotor umfasst mindesten zwei piezoelektrische Stellelemente und einen Rotor, der über die Antriebswelle mit dem Ultraschall-Array gekoppelt ist. Der piezoelektrische Ringmotor bietet Vorteile in Bezug auf einen besonders kompakten und leichten Aufbau der Ultraschall-Messeinrichtung, sowie eine gute Eignung zur Anwendung in einem MRT-Gerät.

Vorteilhafterweise bestehen verschiedene Möglichkeiten, das Ultraschall-Array im Gehäuse einzustellen. Erstens kann vorgesehen sein, dass das Ultraschall-Array um eine Achse parallel zu einer Flächenormalen des Schallfensterabschnitts, d. h. senkrecht zu einem ebenen Schallfensterabschnitt oder parallel zu der Flächenormalen im Zentrum eines gewölbten Schallfensterabschnitts, drehbar ist. In diesem Fall ist die Antriebswelle des elektrischen Motors senkrecht zum Schallfensterabschnitt orientiert. Alternativ kann gemäß einer zweiten Variante das Ultraschall-Array um eine Achse drehbar sein, die relativ zur Flächennormalen des Schallfensterabschnitts geneigt ist, insbesondere senkrecht relativ zur Flächennormalen des Schallfensterabschnitts (d. h. parallel zu einem ebenen Schallfensterabschnitt oder senkrecht zu der Flächenormalen im Zentrum eines gewölbten Schallfensterabschnitts) verläuft. In diesem Fall ist die Antriebswelle des elektrischen Motors relativ zur Flächennormalen des Schallfensterabschnitts geneigt.

Besondere Vorteile für die Kombination der Ultraschall-Messeinrichtung mit einem MRT-Gerät ergeben sich, wenn die Ultraschall-Messeinrichtung, insbesondere das Ultraschall-Array und das Gehäuse aus magnetresonanz-kompatiblen Materialien hergestellt ist. Wenn eine Stelleinrichtung vorgesehen ist, so ist auch diese aus magnetresonanz-kompatiblen Materialien hergestellt. Vorteilhafterweise wird durch einen Ausbau, der aus magnetresonanz-kompatiblen Materialien besteht, eine Störung des Betriebs des MRT-Geräts minimiert oder ausgeschlossen. Magnetresonanz-kompatible Materialien sind Materialien, die in Reaktion auf Hochfrequenz-Felder, die im MRT-Gerät auftreten, keine oder nur vernachlässigbare Magnetresonanz-Signale abgeben. Besonders bevorzugt umfassen die magnetresonanz-kompatiblen Materialien Piezokeramik, wie z. B. PZT (Bleizirkonat-Titanatkeramik), piezoelektrisch aktive Einkristalle oder Kunststofffolien (PVDF, Copolymer), ein Kompositmaterial (z.B. 3-1-PZT-Komposite) aus Kunststoff und Metalloxid- und/oder Metallpulver, insbesondere Aluminiumoxid- und/oder Wolframpulver, , Kupfer, Zink, Leitklebstoff, insbesondere Silberpartikel enthaltend, und/oder Kunststoff, wie z.B. POM (Polyoxymethylen), PEEK (Polyetheretherketon), PU (Polyurethan), Silikon, PET (Polyethylenterephthalat), PC (Polycarbonat) oder Epoxidharz.

Weitere vorteilhafte Modifizierungen der Ultraschall-Messeinrichtung ergeben sich, wenn diese eine Sensoreinrichtung enthält, mit der geometrische Parameter der Ultraschall-Messeinrichtung erfassbar sind. Gemäß einer ersten Variante umfasst die Sensoreinrichtung einen Richtungssensor, mit dem die aktuelle Orientierung des Ultraschall-Arrays in dem Gehäuse, insbesondere relativ zum Schallfensterabschnitt, erfassbar ist. Vorteilhafterweise ermöglicht der Richtungssensor die Erfassung der Empfindlichkeitscharakteristik, ohne dass eine Ultraschallmessung durchgeführt werden muss. Alternativ oder zusätzlich umfasst die Sensoreinrichtung gemäß einer weiteren Variante einen Positionssensor, mit dem die Position der Ultraschall-Messeinrichtung im Raum, insbesondere relativ zu einer weiteren Ultraschall-Messeinrichtung und/oder zum zu untersuchenden Gegenstand und/oder relativ zu einem weiteren Gerät, wie z.B. einem MRT-Gerät erfassbar ist. Der Positionssensor erleichtert die Anwendung der Ultraschall-Messeinrichtung insbesondere bei der multimodalen Bildgebung oder der Kombination mit Radiotherapieverfahren.

Gemäß einer weiteren vorteilhaften Ausführungsform der Offenbarung ist die Ultraschall-Messeinrichtung mit einer abgeschirmten elektrischen Leitungsverbindung ausgestattet, über die das Ultraschall-Array und die Stelleinrichtung mit einer Steuereinrichtung koppelbar ist. Die Leitungsverbindung kann mehrere oder bevorzugt ein einziges Kabel umfassen, wobei die kompletten Funktionen der Ultraschall-Messeinrichtung, insbesondere in Bezug auf die Erzeugung und/oder Erfassung von Ultraschallsignalen und die Einstellung des Ultraschall-Arrays mit der Steuereinrichtung steuerbar sind.

Eine Hauptanwendung der Ultraschall-Messeinrichtung besteht in der Ultraschall-basierten Bildgebung. Bei dieser wird das Ultraschall-Array sowohl als Emitter von Ultraschallwellen als auch als Detektor von Ultraschallwellen verwendet, um aus den dabei erfassten Ultraschallsignalen Bilder des zu untersuchenden Bereichs zu erzeugen. Es ist jedoch nicht zwingend erforderlich, dass die Ultraschallsignale durch ein Einstrahlen von Ultraschallwellen in den Gegenstand erzeugt werden. Alternativ ist z.B. eine photoakustische Bildgebung möglich, bei der Ultraschallsignale erfasst werden, die in Reaktion auf eine Bestrahlung des zu untersuchenden Bereichs mit gepulstem Licht erzeugt werden. Für die Einkopplung des gepulsten Lichtes ist gemäß einer weiteren vorteilhaften Ausführungsform der Erfindung vorgesehen, dass die Ultraschall-Messeinrichtung mit einer Lichtleitereinrichtung und/oder mindestens einer Lichtquelle ausgestattet ist, mit der Licht insbesondere durch den Schallfensterabschnitt und/oder die Gehäusewand auf den zu untersuchenden Gegenstand gerichtet werden kann.

Gemäß einem zweiten allgemeinen Gesichtspunkt wird die oben genannte Aufgabe durch ein Untersuchungsgerät gelöst, das mindestens eine Ultraschall-Messeinrichtung gemäß dem oben genannten ersten Gesichtspunkt aufweist. Vorzugsweise umfasst das Untersuchungsgerät mehrere Ultraschall-Messeinrichtungen. Wenn gemäß einer weiteren bevorzugten Variante die Ultra-schall-Messeinrichtungen mit einer gemeinsamen Steuereinrichtung verbunden sind, ergeben sich Vorteile für den Betrieb der Ultraschall-Messeinrichtungen und die Auswertung der erfassten Ultraschallsignale. Gemäß einer besonders bevorzugten Ausführungsform der Erfindung ist das Untersuchungsgerät für einen Betrieb in Kombination mit einem MR-Tomographen oder Strahlentherapiesystem angeordnet.

Gemäß einem dritten Gesichtspunkt der Offenbarung wird die oben genannte Aufgabe durch ein Verfahren zur Erfassung von Ultraschallsignalen, insbesondere zur Ultraschall-basierten Bildgebung, gelöst, welches die folgenden Schritte umfasst. Erstens wird mindestens eine Ultraschall-Messeinrichtung gemäß dem oben genannten ersten Gesichtspunkt der Erfindung auf der Oberfläche eines zu untersuchenden Gegenstands positioniert. Es ist vorzugsweise eine Fixierung der mindestens einen Ultraschall-Messeinrichtung unter Verwendung des selbstklebenden Schallfensterabschnitts vorgesehen. Wenn eine Stelleinrichtung vorgesehen ist, wird in einem weiteren Schritt das Ultraschall-Array der mindestens einen Ultraschall-Messeinrichtung eingestellt. Das Ultraschall-Array wird so orientiert, dass die Empfindlichkeitscharakteristik in einen gewünschten Bereich des zu untersuchenden Gegenstands gerichtet ist. Des weiteren erfolgt ggf. nach der Einstellung oder während der Bewegung des Ultraschall-Arrays die Betätigung der mindestens einen Ultraschall-Messeinrichtung, z.B. für eine Ultraschall-basierte Bildgebung für einen Emissions- und Empfangsbetrieb aktiviert oder für eine photoakustische Bildgebung für den Empfangsbetrieb aktiviert.

Weitere Einzelheiten und Vorteile der Erfindung werden im Folgenden unter Bezug auf die beigefügten Zeichnungen beschrieben. Es zeigen:
- Figur 1:: eine schematische Phantomdarstellung einer ersten Ausführungsform der erfindungsgemäßen Ultraschall-Messeinrichtung;
- Figuren 2 und 3:: schematische Illustrationen einer weiteren Ausführungsform der erfindungsgemäßen Ultraschall-Messeinrichtung mit vertikaler Drehachse des Ultraschall-Arrays;
- Figur 4:: eine schematische Illustration von Varianten des Schallfensterabschnitts einer erfindungsgemäßen Ultraschall-Messeinrichtung mit Wechselfunktionalität;
- Figuren 5 und 6:: schematische Illustrationen einer weiteren Ausführungsform der erfindungsgemäßen Ultraschall-Messeinrichtung mit horizontaler Drehachse des Ultraschall-Arrays;
- Figur 7:: schematische Illustrationen einer weiteren Ausführungsform der erfindungsgemäßen Ultraschall-Messeinrichtung, die mit einem Richtungssensor ausgestattet ist;
- Figuren 8 und 9:: schematische Illustrationen einer weiteren Ausführungsform der erfindungsgemäßen Ultraschall-Messeinrichtung, die mit einer Lichtleitereinrichtung ausgestattet ist;
- Figur 10:: eine Illustration der Bereitstellung eines Positionssensors in einer erfindungsgemäßen Ultraschall-Messeinrichtung; und
- Figur 11:: eine schematische Illustration der Kombination eines erfindungsgemäßen Untersuchungsgeräts mit einem MRT-Gerät.

Ausführungsformen der Erfindung werden im Folgenden unter beispielhaftem Bezug auf eine Ultraschall-Messeinrichtung beschrieben, die als Ultraschall-Messkopf für die Ultraschall-Bildgebung oder die photoakustische Bildgebung, insbesondere in Kombination mit einem MRT-Gerätausgelegt ist. Es wird betont, dass die Anwendung der Erfindung nicht auf die genannten Beispiele beschränkt, sondern entsprechend bei anderen Formen der multimodalen Bildgebung oder bei anderen Anwendungen von Ultraschall, insbesondere in Kombination mit (Radio)Therapieverfahren, möglich ist. In Abhängigkeit von der konkreten Anwendung der Erfindung kann die Ultraschall-Messeinrichtung bei Bedarf mit einer elektro-magnetischen Abschirmung ausgestattet sein. Die meisten Ausführungsformen der Erfindung zeigen die Ultraschall-Messeinrichtung mit einem im Gehäuse beweglichen Ultraschall-Array und einer Stelleinrichtung. Die Umsetzung der Erfindung ist jedoch nicht auf diese Ausführungsformen beschränkt, sondern entsprechend auch mit einem im Gehäuse starr angeordneten Ultraschall-Array möglich. Einzelheiten des Aufbaus und des Betriebs eines Ultraschall-Arrays, insbesondere für Bildgebungszwecke, und eines MRT-Geräts werden hier nicht beschrieben, da diese an sich aus dem Stand der Technik bekannt sind.

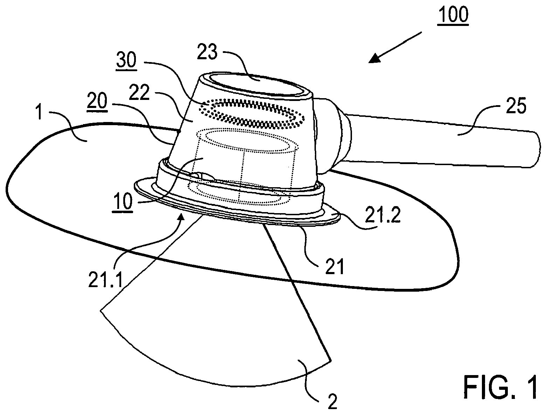

Figur 1 illustriert schematisch eine erste Ausführungsform der Ultraschall-Messeinrichtung 100 in Perspektivansicht. Die Ultraschall-Messeinrichtung 100 umfasst ein Ultraschall-Array 10, das gemeinsam mit einer Stelleinrichtung 30 in einem Gehäuse 20 angeordnet ist. Das Ultraschall-Array 10 und die Stelleinrichtung 30 im Innenraum des Gehäuses 20 sind zu Illustrationszwecken lediglich schematisch gepunktet gezeigt. Weitere Einzelheiten dieser Komponenten werden unten unter Bezug auf die weiteren Figuren beschrieben.

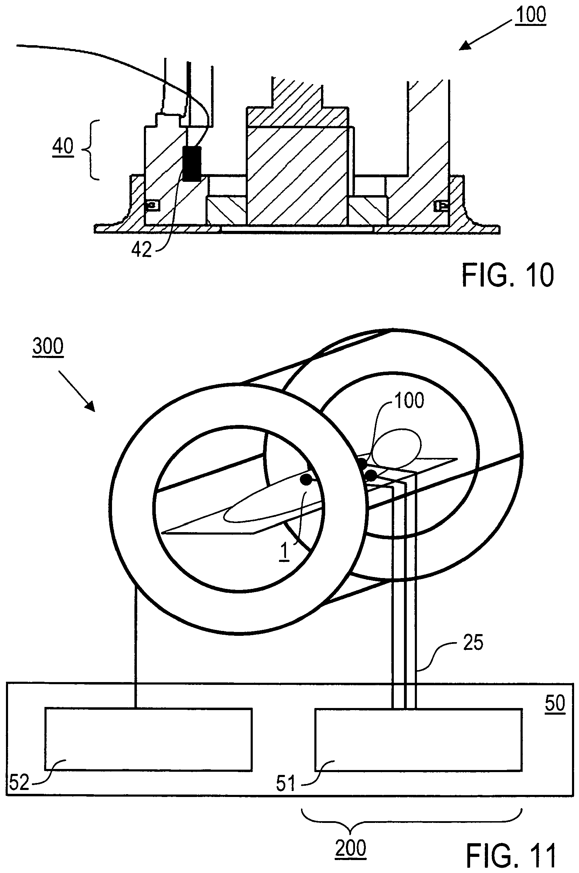

Das Gehäuse 20 umfasst einen Schallfensterabschnitt 21 und eine Gehäusewand 22, 23, mit der eine Verbindungsleitung 25 zur elektrischen Verbindung des Ultraschall-Arrays 10 und der Stelleinrichtung 30 mit einer Steuereinrichtung (nicht gezeigt, siehe Figur 11) gekoppelt ist. Das Gehäuse 20 hat eine Konusform, deren Durchmesser sich vom Schallfensterabschnitt 21 hin zu einem Deckelteil 23 verringert. Das Deckelteil 23 ist ein Teil der Gehäusewand 22, das bei Bedarf, z. B. für Wartungs- oder Kontrollzwecke geöffnet werden kann. Die zum zu untersuchenden Gegenstand 1 weisende Seite der Ultra-schall-Messeinrichtung 100, insbesondere die Seite des Gehäuses 20 mit dem Schallfensterabschnitt 21 wird hier ohne beschränkende Wirkung als Unterseite bezeichnet, während die entgegen gesetzte Seite auch als Oberseite bezeichnet wird. Bei Anwendung der Ultraschall-Messeinrichtung 100 ist es jedoch nicht erforderlich, dass die Unterseite in Gravitationsrichtung weist. Die Gehäusewand 22 ist bei einigen der unten beschriebenen Figuren nicht gezeigt.

Die Ultraschall-Messeinrichtung 100 hat z. B. die folgenden Maße: Durchmesser des Schallfensterabschnitts 21: 4 cm, Durchmesser des Deckelteils 23: 2 cm, Höhe des Gehäuses 20: 2 cm, Masse des Gehäuses 20 mit dem Ultraschall-Array 10 und der Stelleinrichtung 30: 30 g. Die Gehäusewand 22 und das Deckelteil 23 sind z. B. aus Kupfer oder einem mit Kupfer beschichtetem Kunststoff hergestellt.

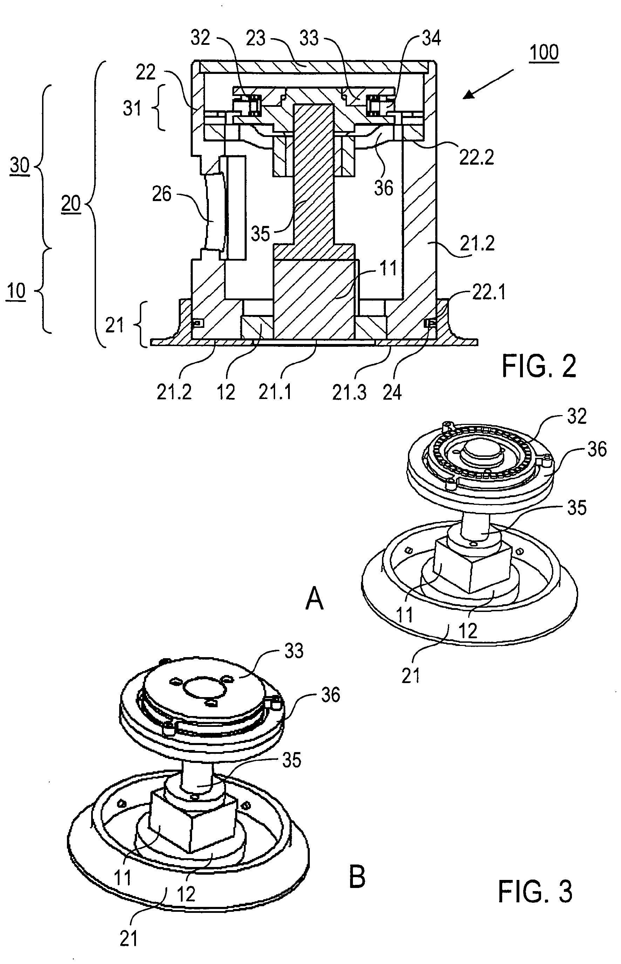

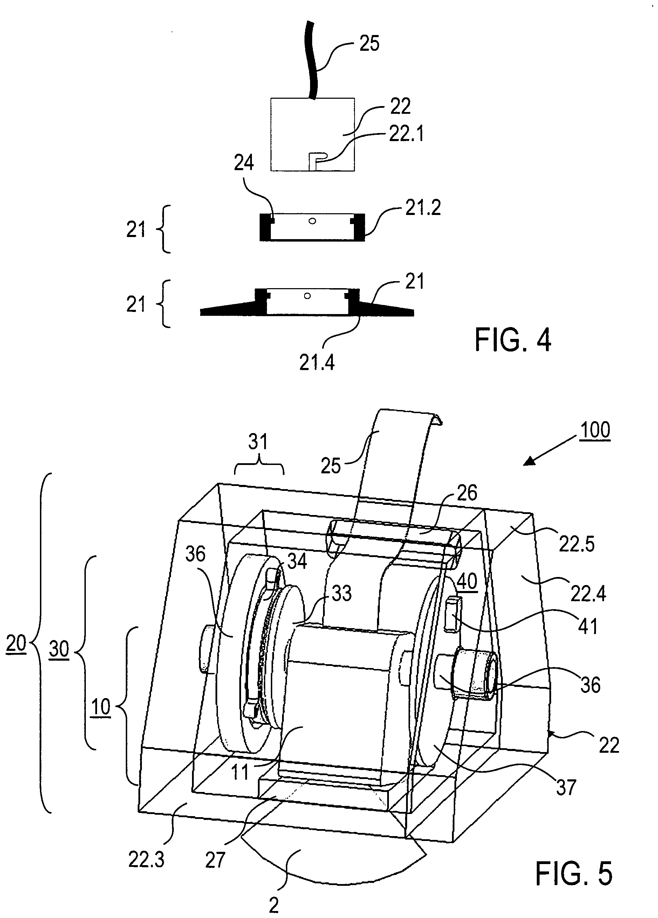

Der Schallfensterabschnitt 21 weist eine ebene Folie 21.1, z. B. aus PEEK, mit einer Dicke von 100 µm auf. Die Folie 21.1 ist in einem Halterahmen 21.2, z. B. aus Kupfer oder einem mit Kupfer beschichtetem Kunststoff, mit einem umlaufenden Vorsprung befestigt, dessen Innendurchmesser an den Außendurchmesser der Gehäusewand 22 angepasst ist. Der Vorsprung trägt als Eingriffselemente 24, die in den Figuren 2 und 4 gezeigt sind. Der Schallfensterabschnitt 21 bildet eine auf die Gehäusewand 22 aufsetzbare Kappe, die bei Bedarf austauschbar ist. Die Folie 21.1 ist z. B. eine selbstklebende Folie des Herstellers 3M, USA.

Wenn eine elektro-magnetische Abschirmung des Innenraums des Gehäuses benötigt wird, ist die Folie 21.1 auf einer Seite z. B. mit einer elektrisch leitenden Schicht (z.B. Sputterschicht aus Aluminium, Kupfer, Gold, Titan, Zink etc.) versehen, die mit der Abschirmung der Gehäusewand 22 über den Halterahmen 21.2 elektrisch verbunden ist. Die elektrisch leitende Schicht ist vorzugsweise auf der Innenseite der Folie 21.1. vorgesehen.

Die Abschirmung umfasst vorzugsweise eine Doppelschirmung des Ultraschall-Arrays 10 und der Stelleinrichtung 30. Der erste, auf Massepotential befindliche Schirm wird zum Beispiel durch die Gehäusewand aus Kupfer und der elektrisch leitenden Schicht gebildet. Der zweite Schirm zur Dämpfung von elektro-magnetischen Feldern aus der Umgebung der Ultraschall-Messeinrichtung wird z. B. durch eine aus Aluminium und/oder Kupfer hergestellte Hülle gebildet, die sich von der Verbindungsleitung 25 zum Ultraschall-Array 10 erstreckt. Des Weiteren ist alternativ oder zusätzlich eine Mantelwellensperre zwischen dem Gehäuse 20 und der Verbindungsleitung 25 vorgesehen.

Auf der Außenseite des Schallfensterabschnitts 21 ist auf der Folie 21.1 und/oder dem Halterahmen 21.2 eine Klebeschicht vorgesehen, mit der die Ultraschall-Messeinrichtung 100 auf einem Körperabschnitt des zu untersuchenden Gegenstands 1 fixierbar ist. Der Gegenstand 1 (in Figur 1 teilweise dargestellt) ist z. B. der Körper eines Probanden in der medizinischen Bildgebung oder ein unter Verwendung von Ultraschall zu untersuchenden Material.

Zur Anwendung der Ultraschall-Messeinrichtung 100 wird diese mit dem Schallfensterabschnitt 21 auf die Oberfläche des zu untersuchenden Gegenstands 1 aufgeklebt. Die Ausrichtung des Ultraschall-Arrays 10 wird mit der Stelleinrichtung 30 eingestellt. Das Ultraschall-Array 10 wird im Innenraum des Gehäuses 20 z. B. so gedreht und/oder translatorisch verschoben, dass das Schallfeld 2 eine vorbestimmte Ausrichtung in einem zu untersuchenden Bereich im Gegenstand 1 hat. Anschließend folgt der Betrieb des Ultraschall-Arrays 10, d. h. die Emission eines Schallfeldes 2 von Ultraschall-Wellen in den zu untersuchenden Bereich und die Detektion von rückreflektierten Ultraschall-Wellen, die Übermittlung der erfassten Ultraschallsignale an die Steuereinrichtung und die Signalverarbeitung zur Erzeugung von Ultraschallbildern.

Alternativ ermöglicht die Ultraschall-Messeinrichtung 100, eine Datenerfassung während der Bewegung des Ultraschall-Arrays 10, z. B. für eine Volumenerfassung oder 3D-Bildgebung. Es kann zum Beispiel vorgesehen sein, dass sich das Ultraschall-Arrays 10 während des Betriebs des Ultra-schall-Arrays 10 zur Datenerfassung permanent dreht.

Bei einer Ausführungsformen mit einem im Gehäuse 20 starr angeordneten Ultraschall-Array 10 ist die Ultraschall-Messeinrichtung 100 aufgebaut, wie oben beschrieben ist, wobei jedoch die Stelleinrichtung 30 durch eine feste Halterung des Ultraschall-Arrays 10 im Gehäuse 20 ersetzt ist.

Weitere Einzelheiten einer Ausführungsform der Ultraschall-Messeinrichtung 100 mit vertikaler Drehachse des Ultraschall-Arrays 10 sind in den Figuren 2 und 3A, 3B gezeigt. Die Figuren 3A, 3B illustrieren die Verbindung aus Stelleinrichtung 30 und Ultraschall-Array 10 ohne die Gehäusewand 22. Gemäß der schematischen Schnittansicht in Figur 2 umfasst das Gehäuse 20 den Schallfensterabschnitt 21 und die Gehäusewand 22 mit dem Deckelteil 23. Die Gehäusewand 22 hat eine zylindrische Form mit einer seitlichen Öffnung 26 zur Ankopplung der Verbindungsleitung (in Figur 2 nicht gezeigt). Die Gehäusewand 22 weist an ihrem zum Schallfensterabschnitt 21 weisenden Rand Ausnehmungen 22.1 zur Aufnahme der Eingriffselemente 24 des Schallfensterabschnitts 21 auf (siehe auch Figur 4). Auf der Innenseite der Gehäusewand 22 ist in einem oberen, zum Deckelteil 23 weisenden Bereich des Innenraums eine umlaufende Abstufung 22.2 vorgesehen, die eine Halterung für die Stelleinrichtung 30 bildet.

Der Schallfensterabschnitt 21 ist über den Halterahmen 21.2 und die Eingriffselemente 24 mit der Gehäusewand 22 verbunden. Der Halterahmen 21.2 trägt auf seiner freiliegenden Oberfläche eine Klebeschicht 21.3, die zur Fixierung der Ultraschall-Messeinrichtung 100 auf dem zu untersuchenden Gegenstand vorgesehen ist.

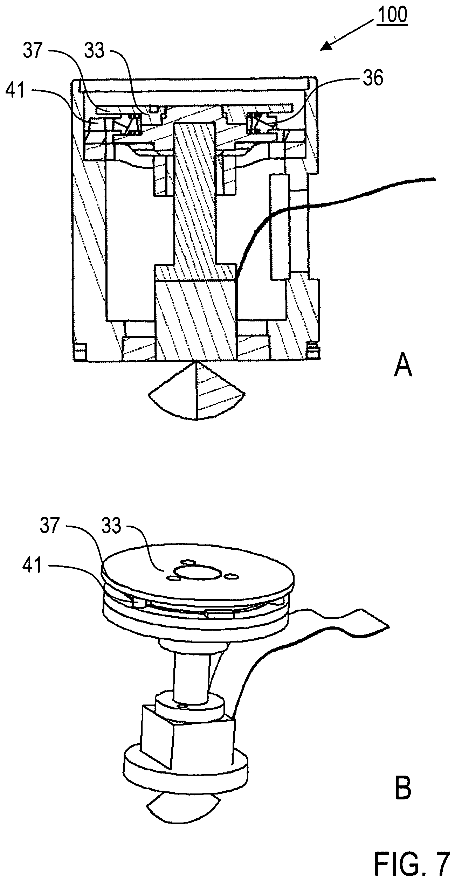

Das Ultraschall-Array 10 umfasst eine Gruppe von Ultraschall-Wandlerelementen 11 und ein Fassungsteil 12. Die Ultraschall-Wandlerelemente 11 umfassen je nach Anwendungsfall eine definierte Anzahl von Einzelelementen und Anordnungen bei einer definierten geometrischen Größe und Arbeitsfrequenz. Das Fassungsteil 12 hat z. B. die Gestalt einer zylindrischen Scheibe (siehe Figur 3A, 3B), in welche die Ultraschall-Wandlerelemente 11 eingelassen sind. Das Fassungsteil 12 hat eine Doppelfunktion. Erstens bildet eine Oberfläche des Fassungsteils 12 mit der Abstrahlseite der Ultraschall-Wandlerelemente 11 eine Auflagefläche. Im zusammengesetzten Zustand berührt die Innenseite des Schallfensterabschnitts 21 die Auflagefläche des Ultraschall-Arrays 10. Zur verbesserten Übertragung von Ultraschallwellen kann zwischen dem Ultra-schall-Array 10 und dem Schallfensterabschnitt 21 ein Übertragungsmedium, wie z. B. ein Gel, Öl oder Fett, vorgesehen sein. Bei Bedarf kann das Übertragungsmedium, z. B. bei einem Wechsel des Schallfensterabschnitts 21 ausgetauscht oder erneuert werden. Zweitens bildet das Fassungsteil 12 einen Befestigungspunkt für weitere Bauteile, wie z. B. Lichtleiter einer Lichtleitereinrichtung (siehe Figur 7).

Die Stelleinrichtung 30 ist im Gehäuse 20 auf der Abstufung 22.2 gehaltert. Die Stelleinrichtung 30 umfasst einen piezoelektrischen Ringmotor 31 mit Piezoaktoren 32, einem Rotor 33, einem Stator 34, einer Antriebswelle 35 und einer Lagerung 36. Die Antriebswelle 35 ist an ihrem ersten Ende mit dem Rotor 33 und an ihrem zweiten Ende mit dem Ultraschall-Array 10, insbesondere den Ultraschall-Wandlerelementen 11, verbunden. Die Ultraschall-Wandlerelemente 11 sind an der Antriebswelle 35 z. B. angeklebt oder angeschraubt.

Die Piezoaktoren 32 sind ringförmig angeordnet. Bei Beaufschlagung der Piezoaktoren 32 mit einer Anregungsspannung kann eine Drehung des Rotors 33 mit der Antriebswelle 35 und dem Ultraschall-Array 10 um die vertikale Achse (z-Achse) bewirkt werden. Wie in Figur 3 gezeigt ist, bilden die Piezoaktoren 32 zwei Gruppen, die einerseits ringförmig auf dem Stator 34 (Figur 3A) und andererseits ringförmig auf dem Rotor 33 (Figur 2, Figur 3B) angeordnet sind. Es ist nicht zwingend vorgesehen, dass die Piezoaktoren 32 gleichmäßig entlang des gesamten Umfangs des Rotors 33 und des Stators 34 angeordnet sind. In Abhängigkeit von der Anwendung der Erfindung kann es ausreichend sein, einzelne Piezoaktoren für schrittweise Verstellungen des Ultraschall-Arrays 10 vorzusehen.

Abweichend von der Illustration kann die Stelleinrichtung 30 für eine translatorische Bewegung ausgelegt sein und einen piezoelektrischen Linearantrieb aufweisen (nicht dargestellt).

In Figur 4 sind verschiedene Varianten des Schallfensterabschnitts 21 schematisch illustriert. Beispielsweise kann der Halterahmen 21.2 auf den umlaufenden Vorsprung beschränkt sein, auf dessen Innenseite die Eingriffselemente 24 vorgesehen sind. Alternativ kann der Halterahmen 21.2 sich in radialer Richtung erstrecken, um eine kreisringförmige Klebefläche 21.4 zu bilden.

Die Aufnahmen 22.1 auf der Außenseite der Gehäusewand 22 umfassen eine abgewinkelte Nut zur Aufnahme der Eingriffselemente 24. Zur Befestigung des Schallfensterabschnitts 21 an der Gehäusewand 22 wird der Halterahmen 21.2 auf die Gehäusewand 22 aufgeschoben, so dass die Eingriffselemente 24 in die Aufnahmen 22.1 gelangen. Durch ein Verdrehen des Schallfensterabschnitts 21 und der Gehäusewand 22 relativ zu einander wird der Schallfensterabschnitt 21 an der Gehäusewand 22 verankert.

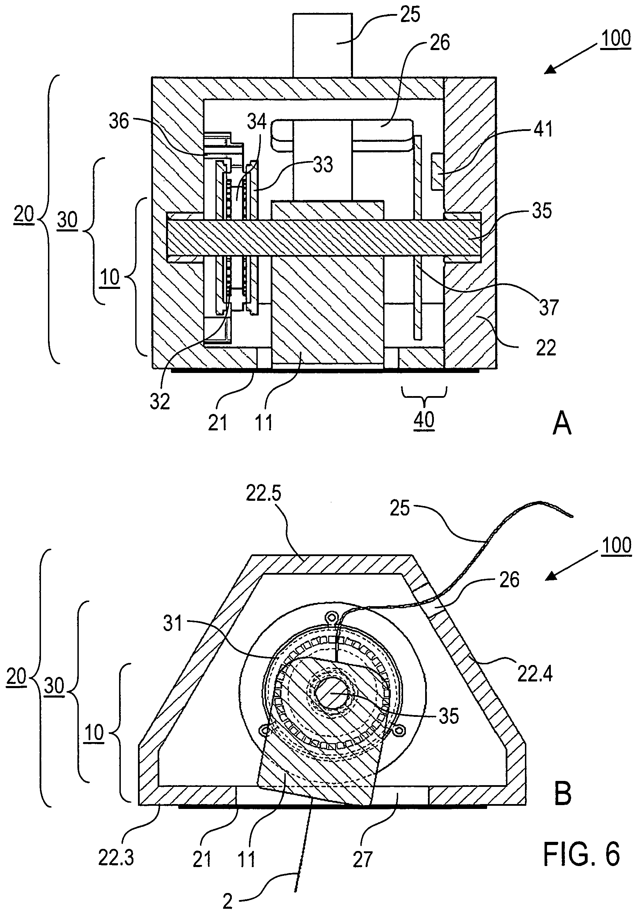

Die Figuren 5 und 6 illustrieren eine abgewandelte Ausführungsform der erfindungsgemäßen Ultraschall-Messeinrichtung 100 mit einer horizontal ausgerichteten Drehachse des Ultra-schall-Arrays 10. Figur 5 zeigt eine Perspektiv-Phantomansicht, während die Figuren 6A und 6B Schnittansichten parallel und senkrecht zur Drehachse zeigen. Bei dieser Ausführungsform der Erfindung umfasst die Gehäusewand 22 des Gehäuses 20 eine ebene Grundfläche 22.3, ebene und abgewinkelte Seitenflächen 22.4 und eine Deckfläche 22.5. Der Schallfensterabschnitt 21 ist mit der Grundfläche 22.3 verbunden, z. B. in diese eingelassen oder auf diese aufgesetzt (siehe Figur 6B). In einer der Seitenflächen 22.4 ist eine Öffnung 26 zur Durchführung oder Ankopplung einer Verbindungsleitung 25 vorgesehen. Mit der Öffnung 27 in der Grundfläche 22.3 hin zum Schallfensterabschnitt 21 wird eine Ausnehmung gebildet, in die Teile des Ultraschall-Arrays 10 beim Verschwenken hineinragen. Zur Verbesserung der Schallkopplung kann die Ausnehmung in der Öffnung 27 mit einem Übertragungsmedium, z.B. einem Gel, Fett oder Öl gefüllt sein.

Die Stelleinrichtung 30 umfasst, wie oben unter Bezug auf die Figuren 2 und 3 beschrieben ist, einen piezoelektrischen Ringmotor 31 mit Piezoaktoren 32, einem Rotor 33, einem Stator 34, einer Antriebswelle 35 und einer Lagerung 36. Der piezoelektrische Ringmotor 31 ist so ausgerichtet, dass die Drehachse der Antriebswelle 35 parallel zur Grundfläche 22.3 verläuft. Die Antriebswelle 35 ist auf den zueinander entgegengesetzten Innenseiten der Seitenflächen 22.4 drehbar gelagert. Auf der Antriebswelle 35 ist das Ultraschall-Array 10 befestigt, das bei Betätigung des piezoelektrischen Ringmotors 31 um die Drehachse verschwenkbar ist (Figur 6B), so dass das Schallfeld 2 in verschiedene Richtungen im zu untersuchenden Gegenstand weist.

Die Figuren 5 und 6A zeigen am Ende der Antriebswelle 35 eine Sensoreinrichtung 40 zur Erfassung geometrischer Parameter der Ultraschall-Messeinrichtung 100, insbesondere des Ultra-schall-Arrays 10. Bei der dargestellten Ausführungsform umfasst die Sensoreinrichtung 40 einen optischen Richtungssensor 41, der auf der Innenseite der Seitenfläche 22.4 angeordnet ist. Auf der Antriebswelle 35 sitzt eine mit einem Muster versehene Taktscheibe 37, deren Position mit dem Richtungssensor 41 erfasst wird.

Der Richtungssensor 41 kann auch bei der Ausführungsform der Ultraschall-Messeinrichtung 100 mit vertikaler Drehachse vorgesehen sein, wie schematisch in den Figuren 7A und 7B gezeigt ist. In diesem Fall ist die Taktscheibe 37 mit dem Rotor 33 verbunden, während der Richtungssensor 41 z. B. auf dem Stator 34 sitzt.

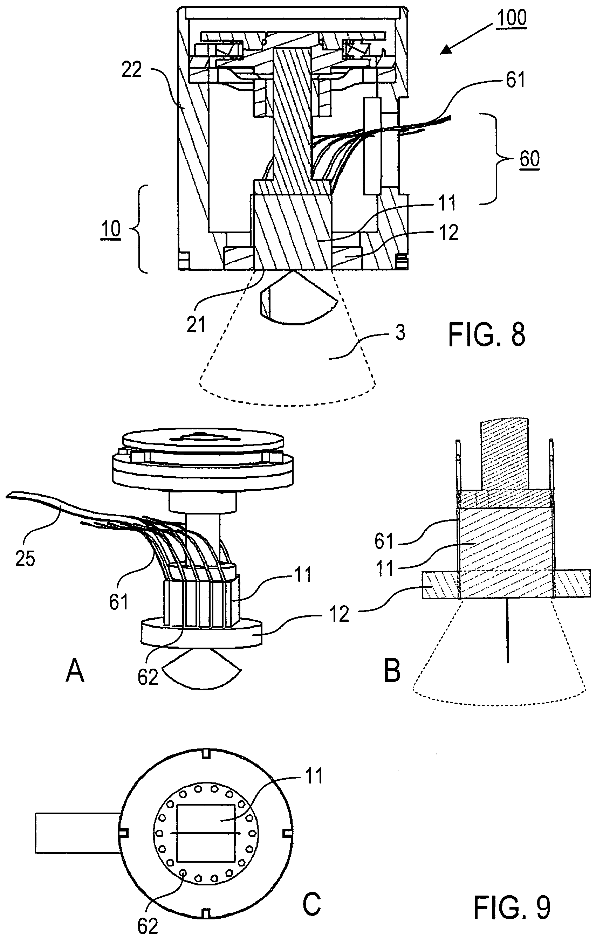

Die Figuren 8 und 9 illustrieren eine Ausführungsform der erfindungsgemäßen Ultraschall-Messeinrichtung 100, die für die photoakustische Bildgebung ausgelegt ist. In diesem Fall ist eine Lichtleitereinrichtung 60, umfassend eine Vielzahl von Lichtleitern 61, vorgesehen, die z. B. als Teil der Verbindungsleitung 25 oder zusätzlich zu dieser zum Ultraschall-Array 10 geführt sind. Die Endabschnitte der Lichtleiter 61 sind so auf den Ultraschall-Wandlerelementen 11 und/oder im Fassungsteil 12 fixiert, dass die Austrittsenden 62 der Lichtleiter 61 zum Schallfensterabschnitt 21 weisen. Typischerweise ist eine Vielzahl von Lichtleitern 61 vorgesehen, die ein Muster mit mehreren geraden Reihen von punktförmigen Anregungslichtquellen (schematische Perspektivansicht in Figur 9A, Schnittansicht in Figur 9B), ein rechteckiges Muster oder ein kreisförmiges Muster (Draufsicht in Figur 9C) von punktförmigen Anregungslichtquellen bilden. Die Lichtleiter können mit optischen Elementen, z. B. optischen Linsen, ausgestattet sein, z. B. um Anregungslicht zu fokussieren. Alternativ könnten die Lichtleiter an der Gehäusewand 22 fixiert sein. Die Lichtleiter 61 können auch so angeordnet sein, dass die Austrittsenden 62 zur Außenseite des Gehäuses 20 frei liegen. Über die Lichtleiter 61 wird gepulstes Anregungslicht 3 in den zu untersuchenden Gegenstand eingekoppelt. Die in Reaktion auf die Anregung im zu untersuchenden Gegenstand erzeugten mechanischen Schwingungen werden als Ultraschallschwingungen mit dem Ultraschall-Array 10 erfasst.

Abweichend von der Illustration können Lichtquellen, wie z. B. LED's, im und/oder außen am Gehäuse 20 angeordnet sein, um Anregungslicht zur Einkopplung in den zu untersuchenden Gegenstand zu erzeugen (nicht dargestellt).

Die Sensoreinrichtung 40 kann gemäß einer weiteren Variante der Erfindung einen Positionssensor 42 umfassen, der schematisch in Figur 10 gezeigt ist. Mit dem Positionssensor 42, der im Inneren integriert oder abweichend von der Illustration in Figur 10 auf der Außenseite angebracht sein kann, ist die Raumposition der Ultraschall-Messeinrichtung 100, z. B. relativ zu einem angrenzenden MRT-Gerät oder zu einer Halteplattform für den zu untersuchenden Gegenstand erfassbar. Der Positionssensor 42 ist z. B. für die Erfassung von gepulsten Hochfrequenzsignalen einer Gruppe von Antennen in der Umgebung der Ultraschall-Messeinrichtung 100 eingerichtet. Nach einer Kalibrierung können alle Positionen von Ultraschall-Messeinrichtungen in einem globalen Referenzsystem erfasst und mit den geometrischen Parametern weiterer Geräte abgestimmt werden.

Figur 11 illustriert schematisch eine Ausführungsform eines erfindungsgemäßen Untersuchungsgeräts 200 mit einer Vielzahl von Ultraschall-Messeinrichtungen 100, die zur gleichzeitigen oder sequentiellen Ultraschallbildgebung an einem Probanden 1 angeordnet sind. Der Proband 1 befindet sich in einem MR-Tomographen 300. Die Ultraschall-Messeinrichtungen 100 sind über Verbindungsleitungen 25 mit einer Steuereinrichtung 50 verbunden. Die Steuereinrichtung 50 enthält eine erste Betriebsschaltung 51 zum Betrieb der Ultraschall-Messeinrichtungen 100 und eine zweite Betriebsschaltung 52 zum Betrieb des MR-Tomographen 300. Die mit dem Untersuchungsgerät 200 und dem MR-Tomographen 300 ermittelten Bilder können miteinander registriert und einer weiteren Auswertung, Bildverarbeitung und/oder Anzeige unterzogen werden. Es ist nicht zwingend erforderlich, dass die ersten und zweiten Betriebsschaltungen 51, 52 in einer gemeinsamen Steuereinrichtung 50 verbunden sind. Das Untersuchungsgerät 200 und der MR-Tomograph 300 können mit separaten Betriebsschaltungen verbunden sein.

Die in der Beschreibung, den Zeichnungen und den Ansprüchen offenbarten Merkmale der Erfindung können sowohl einzeln als auch in Kombination für die Verwirklichung der Erfindung in ihren verschiedenen Ausgestaltungen von Bedeutung sein.

## Patentansprüche

1. Ultraschall-Messeinrichtung (100), umfassend:
- ein Ultraschall-Array (10), das zur Erfassung von Ultraschallsignalen konfiguriert ist, und
- ein Gehäuse (20), das einen Schallfensterabschnitt (21) und eine Gehäusewand (22) umfasst, wobei das Ultraschall-Array (10) in dem Gehäuse (20) in akustischem Kontakt mit dem Schallfensterabschnitt (21) angeordnet ist, wobei
- der Schallfensterabschnitt (21) für ein Anhaften auf einer Oberfläche des zu untersuchenden Gegenstands (1) konfiguriert ist,
**dadurch gekennzeichnet, dass**
- das Gehäuse (20) eine elektro-magnetische Abschirmung bildet, die gegen Felder in einem Magnetresonanz-Tomographie-Gerät wirksam ist, und
- die Ultraschall-Messeinrichtung (100) eine Stelleinrichtung (30) aufweist, die zur Einstellung und/oder Bewegung des Ultraschall-Arrays (10) relativ zu einem zu untersuchenden Gegenstand (1) eingerichtet ist, wobei
- die Stelleinrichtung (30) einen piezoelektrischen Ringmotor (31) umfasst, mit dem das Ultraschall-Array (10) relativ zum Schallfensterabschnitt (21) beweglich ist, und
- das Ultraschall-Array (10), das Gehäuse (20) und der piezoelektrische Ringmotor (31) aus magnetresonanz-kompatiblen Materialien hergestellt ist, die in Reaktion auf Hochfrequenz-Felder, die im Magnetresonanz-Tomographie-Gerät auftreten, keine oder nur vernachlässigbare Magnetresonanz-Signale erzeugen.

2. Ultraschall-Messeinrichtung gemäß Anspruch 1, bei der der Schallfensterabschnitt (21) mindestens eines der Merkmale aufweist
- der Schallfensterabschnitt (21) ist vom übrigen Gehäuse (20) trennbar angeordnet,
- der Schallfensterabschnitt (21) ist ein Einwegprodukt,
- der Schallfensterabschnitt (21) umfasst eine Kunststofffolie, die insbesondere mit einem Abschirmmaterial versehen ist, und
- der Schallfensterabschnitt (21) ist mit einer selbstklebenden Folie versehen.

3. Ultraschall-Messeinrichtung gemäß Anspruch 2, bei der
- der Schallfensterabschnitt (21) die Kunststofffolie umfasst, die mit dem Abschirmmaterial versehen ist, wobei
- das Abschirmmaterial eine elektrisch leitenden Schicht, insbesondere eine Sputterschicht, umfasst, oder aus leitfähigem Material besteht.

4. Ultraschall-Messeinrichtung gemäß einem der Ansprüche 2 oder 3, bei der
- der Schallfensterabschnitt (21) über Eingriffselemente (24) an der Gehäusewand (22) lösbar befestigt ist.

5. Ultraschall-Messeinrichtung gemäß einem der vorhergehenden Ansprüche, bei der
- das Ultraschall-Array (10) mit der Stelleinrichtung (30) relativ zum Schallfensterabschnitt (21) beweglich ist, und
- die Stelleinrichtung (30) vom Gehäuse (20) eingeschlossen ist.

6. Ultraschall-Messeinrichtung gemäß einem der vorhergehenden Ansprüche, wobei die elektro-magnetische Abschirmung durch mindestens eine der Maßnahmen gebildet wird, die umfassen
- Bildung einer Doppelschirmung des Ultraschall-Arrays (10) und der Stelleinrichtung (30), und
- Bildung einer Mantelwellensperre an einer mit dem Gehäuse (20) gekoppelten Leitungsverbindung (25).

7. Ultraschall-Messeinrichtung gemäß einem der vorhergehenden Ansprüche, bei der
- das Ultraschall-Array (10) um eine Achse parallel zu einer Flächennormalen des Schallfensterabschnitts (21) drehbar ist, und/oder
- das Ultraschall-Array (10) um eine Achse drehbar ist, die relativ zu einer Flächennormalen des Schallfensterabschnitts (21) geneigt ist.

8. Ultraschall-Messeinrichtung gemäß einem der vorhergehenden Ansprüche, bei der die magnetresonanz-kompatiblen Materialien mindestens eines umfassen von:
- Piezokeramik,
- piezoelektrisch aktive Einkristalle,
- piezoelektrisch aktive Polymere,
- Kompositmaterialien aus piezoelektrisch aktiven Einkristallen und Polymeren, insbesondere 3-1-PZT-Komposite,
- Kompositmaterial aus Kunststoff und Metalloxid- oder Metallpulver, insbesondere Aluminiumoxid- oder Wolframpulver,
- Metall, insbesondere Kupfer, Zink, Gold, Titan, oder Aluminium,
- Leitklebstoff, insbesondere mit Silber- oder Goldpartikeln, und
- Kunststoff, insbesondere POM, PEEK, PU, Silikon, Epoxidharz.

9. Ultraschall-Messeinrichtung gemäß einem der vorhergehenden Ansprüche, die mit einer Sensoreinrichtung (40) ausgestattet ist, die mindestens eines umfasst von
- einem Richtungssensor (41), mit dem die Orientierung des Ultraschall-Arrays (10) in dem Gehäuse (20) erfassbar ist, und
- einem Positionssensor (42), mit dem eine Raumposition der Ultraschall-Messeinrichtung (100) erfassbar ist.

10. Untersuchungsgerät (200), das mindestens eine Ultra-schall-Messeinrichtung (100) gemäß einem der vorhergehenden Ansprüche aufweist.

11. Untersuchungsgerät gemäß Anspruch 10, das
- in Verbindung mit einem Magnetresonanz-Tomographie-Gerät (300) angeordnet ist.

12. Verfahren zur Ultraschall-Signalerfassung, insbesondere zur Ultraschall-basierten Bildgebung, mit den Schritten:
- Positionierung und/oder kontinuierliche Bewegung, insbesondere zum Scannen für eine 3D-Datenerfassung, von mindestens einer Ultraschall-Messeinrichtung (100) gemäß einem der Ansprüche 1 bis 9 auf der Oberfläche eines zu untersuchenden Gegenstands (1), und
- Betätigung der mindestens einen Ultraschall-Messeinrichtung (100).

13. Verfahren gemäß Anspruch 12, bei dem
- der zu untersuchende Gegenstand (1) in einem Magnetresonanz-Tomographie-Gerät (300) angeordnet ist, und
- die Ultraschall-basierte Bildgebung während des Betriebs des Magnetresonanz-Tomographie-Gerätes (300) erfolgt.

## Claims

1. Ultrasonic measuring device (100), comprising:
- an ultrasonic array (10) configured to detect ultrasonic signals, and
- a housing (20) comprising an acoustic window portion (21) and a housing wall (22), whereby the ultrasonic array (10) is arranged in the housing (20) in acoustic contact with the acoustic window portion (21), wherein
- the acoustic window portion (21) is configured for an adherence to a surface of the object (1) to be examined,
**characterised in that**
- the housing (20) forms an electromagnetic shielding which is effective against fields in a magnetic resonance tomography device, and
- the ultrasonic measuring device (100) has an actuator device (30) which is configured for the adjustment and/or movement of the ultrasonic array (10) relative to an object to be examined (1), wherein
- the actuator device (30) comprises a piezoelectric ring motor (31) using which the ultrasonic array (10) can be moved relative to the acoustic window portion (21), and
- the ultrasonic array (10), the housing (20) and the piezoelectric ring motor (31) are made of magnetic-resonance-compatible materials, which do not emit or emit only negligible magnetic-resonance signals in response to high-frequency fields occurring in the magnetic resonance tomography device

2. Ultrasonic measuring device in accordance with claim 1, in which the acoustic window portion (21) has at least one of the features
- the acoustic window portion (21) is arranged detachably from the remaining housing (20),
- the acoustic window portion (21) is a disposable product,
- the acoustic window portion (21) comprises a plastic film which, in particular, is provided with a screening material, and
- the acoustic window portion (21) has a self-adhesive film.

3. Ultrasonic measuring device in accordance with claim 2, in which
- the acoustic window portion (21) comprises the plastic film which is provided with the screening material, wherein
- the screening material comprises an electrically conductive layer, in particular a sputter layer, or is made of conductive material.

4. Ultrasonic measuring device in accordance with one of the claims 2 or 3, in which
- the acoustic window portion (21) is detachably attached via grip elements (24) to the housing wall (22).

5. Ultrasonic measuring device in accordance with one of the foregoing claims, in which
- the ultrasonic array (10) can be moved using the actuator device (30) relative to the acoustic window portion (21), and
- the actuator device (30) is enclosed by the housing (20).

6. Ultrasonic measuring device in accordance with one of the foregoing claims, wherein the electromagnetic shielding is formed by at least one of the measures which comprise
- creation of a dual screening of the ultrasonic array (10) and of the actuator device (30), and
- creation of a balun at a line connection (25) coupled with the housing (20).

7. Ultrasonic measuring device in accordance with one of the foregoing claims, in which
- the ultrasonic array (10) can be rotated around an axis parallel to a surface normal of the acoustic window portion (21), and/or
- the ultrasonic array (10) can be rotated around an axis which is inclined relative to a surface normal of the acoustic window portion (21).

8. Ultrasonic measuring device in accordance with one of the foregoing claims, in which the magnetic-resonance-compatible materials comprise at least one of the following:
- piezoceramics,
- piezoelectrically active monocrystals,
- piezoelectrically active polymers,
- composite materials made of piezoelectrically active monocrystals and polymers, in particular 3-1-PZT composites,
- composite material made of plastic and metallic oxide or metallic powder, in particular aluminium oxide or tungsten powder,
- metal, in particular copper, zinc, gold, titanium or aluminium,
- conductive adhesive, in particular with silver or gold particles, and
- plastic, in particular POM, PEEK, PU, silicon, epoxy resin.

9. Ultrasonic measuring device in accordance with one of the foregoing claims, which is provided with a sensor device (40), which comprises at least one of the following:
- a directional sensor (41) using which the alignment of the ultrasonic array (10) in the housing (20) can be detected, and
- a position sensor (42) using which a spatial position of the ultrasonic measuring device (100) can be detected.

10. Examination apparatus (200), which has at least one ultrasonic measuring device (100) in accordance with one of the foregoing claims.

11. Examination apparatus in accordance with claim 15, which
- is arranged in connection with a magnetic resonance tomography device (300).

12. Method for ultrasonic signal detection, in particular for ultrasound-based imaging, with the steps of:
- positioning and/or continuous movement, in particular for scanning for a 3D data detection, of at least one ultrasonic measuring device (100) in accordance with one of the claims 1 to 9 on the surface of an object to be examined (1), and
- actuating of the at least one ultrasonic measuring device (100).

13. Method in accordance with claim 12, in which
- the object to be examined (1) is arranged in a magnetic resonance tomography device (300), and
- the ultrasound-based imaging is conducted during the operation of the magnetic resonance tomography device (300).

## Revendications

1. Dispositif de mesure à ultrasons (100), comprenant :
- un réseau à ultrasons (10), qui est configuré pour détecter des signaux ultrasonores, et
- un boîtier (20), qui comprend une section de fenêtre sonore (21) et une paroi de boîtier (22), dans lequel le réseau à ultrasons (10) est disposé dans le boîtier (20) en contact acoustique avec la section de fenêtre sonore (21), dans lequel
- la section de fenêtre sonore (21) est configurée pour adhérer sur une surface de l'objet (1) à examiner,
**caractérisé en ce que**
- le boîtier (20) forme un écran de protection électromagnétique, qui est efficace contre des champs dans un appareil de tomographie par résonnance magnétique, et
- le dispositif de mesure à ultrasons (100) présente un dispositif de réglage (30), qui est mis au point pour l'ajustement et/ou le déplacement du réseau à ultrasons (10) par rapport à un objet (1) à examiner, dans lequel
- le dispositif de réglage (30) comprend un moteur annulaire (31) piézoélectrique, avec lequel le réseau à ultrasons (10) est mobile par rapport à la section de fenêtre sonore (21), et
- le réseau à ultrasons (10), le boîtier (20) et le moteur annulaire (31) piézoélectrique sont fabriqués à partir de matériaux compatibles avec la résonnance magnétique, qui, en réaction à des champs à fréquence élevée, qui apparaissent dans l'appareil de tomographie par résonnance magnétique, génère des signaux de résonnance magnétique nuls ou seulement négligeables.

2. Dispositif de mesure à ultrasons selon la revendication 1, dans lequel la section de fenêtre sonore (21) présente au moins une des caractéristiques suivantes :
- la section de fenêtre sonore (21) est disposée de manière à pouvoir être séparée du reste du boîtier (20),
- la section de fenêtre sonore (21) est un produit à usage unique,
- la section de fenêtre sonore (21) comprend un film en matière plastique, qui est pourvu en particulier d'un matériau faisant écran de protection, et
- la section de fenêtre sonore (21) est pourvue d'un film autocollant.

3. Dispositif de mesure à ultrasons selon la revendication 2, dans lequel
- la section de fenêtre sonore (21) comprend le film en matière plastique, qui est pourvue du matériau formant écran de protection, dans lequel
- le matériau formant écran de protection comprend une couche électriquement conductrice, en particulier une couche de pulvérisation, ou est constitué d'un matériau conducteur.

4. Dispositif de mesure à ultrasons selon l'une quelconque des revendications 2 ou 3, dans lequel
- la section de fenêtre sonore (21) est fixée de manière amovible au niveau de la paroi de boîtier (22) par l'intermédiaire d'éléments de prise (24).

5. Dispositif de mesure à ultrasons selon l'une quelconque des revendications précédentes, dans lequel
- le réseau à ultrasons (10) est mobile par rapport à la section de fenêtre sonore (21) avec le dispositif de réglage (30), et
- le dispositif de réglage (30) est renfermé par le boîtier (20).

6. Dispositif de mesure par ultrasons selon l'une quelconque des revendications précédentes, dans lequel l'écran de protection électromagnétique est formé par au moins une des mesures qui comprennent :
- la formation d'un double blindage du réseau à ultrasons (10) et du dispositif de réglage (30), et
- la formation d'une barrière d'arbre d'enveloppe au niveau d'un raccordement de conduite (25) couplé au boîtier (20).

7. Dispositif de mesure à ultrasons selon l'une quelconque des revendications précédentes, dans lequel
- le réseau à ultrasons (10) peut être tourné autour d'un axe de manière parallèle par rapport à une normale de surface de la section de fenêtre sonore (21), et/ou
- le réseau à ultrasons (10) peut tourner autour d'un axe, qui est incliné par rapport à une normale de surface de la section de fenêtre sonore (21).

8. Dispositif de mesure à ultrasons selon l'une quelconque des revendications précédentes, dans lequel les matériaux compatibles à résonnance magnétique comprennent au moins un matériau parmi les matériaux qui suivant :
- une piézocéramique ;
- des monocristaux actifs piézoélectriquement ;
- des polymères actifs piézoélectriquement ;
- des matériaux composites composés de monocristaux actifs piézoélectriquement et de polymères, en particulier des composites 3-1-PZT ;
- un matériau composite composé de matière plastique et de poudre d'oxyde métallique ou de poudre de métal, en particulier de poudre d'oxyde d'aluminium ou de poudre de tungstène ;
- un métal, en particulier du cuivre, du zinc, de l'or, du titane ou de l'aluminium ;
- de la colle conductrice, en particulier comprenant des particules d'argent ou d'or ; et
- une matière plastique, en particulier du POM, du PEEK, du PU, de la silicone, de la résine époxy.

9. Dispositif de mesure à ultrasons selon l'une quelconque des revendications précédentes, qui est configuré avec un dispositif de capteur (40), qui comprend au moins un élément parmi les éléments suivantes :
- un capteur de direction (41), avec lequel l'orientation du réseau à ultrasons (10) peut être détectée dans le boîtier (20) ; et
- un capteur de position (42), avec lequel une position spatiale du dispositif de mesure à ultrasons (100) peut être détectée.

10. Appareil d'examen (200), qui présente au moins un dispositif de mesure à ultrasons (100) selon l'une quelconque des revendications précédentes.

11. Appareil d'examen selon la revendication 10, qui est disposé en liaison avec un appareil de tomographie par résonnance magnétique (300).

12. Procédé servant à détecter des signaux ultrasonores, en particulier servant à l'imagerie basée sur les ultrasons, comprenant les étapes consistant à :
- positionner et/ou déplacer en continu, en particulier aux fins du balayage pour une détection de données 3D, au moins un dispositif de mesure à ultrasons (100) selon l'une quelconque des revendications 1 à 9 sur la surface d'un objet (1) à examiner ; et
- actionner l'au moins un dispositif de mesure à ultrasons (100).

13. Procédé selon la revendication 12, dans lequel
- l'objet (1) à examiner est disposé dans un appareil de tomographie par résonnance magnétique (300), et
- l'imagerie basée sur les ultrasons est réalisée au cours du fonctionnement de l'appareil de tomographie par résonnance magnétique (300).
